# FASCICULE DE BREVET EUROPEEN

(11) **EP 2 555 697 B1**
(45) Date de publication et mention de la délivrance du brevet: **30.03.2016**
(21) Numéro de dépôt: 11719595.8
(22) Date de dépôt: 05.04.2011
(51) Int. Cl.: A61B 17/70

(54) **SYSTEME ET DISPOSITIF DE LIAISON TRANSVERSE POUR COLONNE VERTEBRALE**
QUERLAUFENDES VERBINDUNGSSYSTEM UND VORRICHTUNG FÜR DIE WIRBELSÄULE
TRANSVERSE CONNECTION SYSTEM AND DEVICE FOR THE VERTEBRAL COLUMN

(30) Priorité: 08.04.2010 FR 1001489
(43) Date de publication de la demande: 13.02.2013
(73) Titulaire: Implanet, 33650 Martillac (FR)
(72) Inventeur: LE COUËDIC, Régis, F-33800 Bordeaux (FR); BACCELLI, Christian, F-33650 Saucats (FR)
(74) Mandataire: Benech, Frédéric
(86) Numéro de dépôt international: PCT/FR2011/000200
(87) Numéro de publication internationale: WO 2011/124789

(56) Documents cités:
- US-A1- 2005 090 821
- US-A1- 2005 228 377
- US-A1- 2007 043 365
- US-A1- 2007 276 367
- US-A1- 2008 109 039
- US-A1- 2008 306 538

## Description

La présente invention concerne un système de liaison entre deux tiges cylindriques longitudinales de maintien rachidien, comportant au moins un élément allongé transversal muni à chacune de ses extrémités d'une tête de fixation sur une tige correspondante.

Elle concerne également un dispositif comprenant un tel système.

Elle trouve une application particulièrement importante bien que non exclusive dans le domaine de la réparation de la colonne vertébrale du fait de traumatismes liés à un accident, ou encore pour compenser une malformation comme une scoliose ou toute autre déformation, qu'elle soit de naissance ou acquise.

Il est classique dans de tels cas de mettre en place un dispositif implantable de redressement et/ou consolidation utilisant deux tiges disposées sensiblement parallèlement à la colonne vertébrale de part et d'autre de cette dernière.

Chaque tige est fixée à au moins deux vertèbres par exemple par des vis vissées dans une vertèbre correspondante en vis à vis, ou encore par le biais de crochets.

Chaque vis ou crochet comporte une partie de fixation réglable en position sur la tige, la distance entre les points de fixation sur les vertèbres adjacentes étant prévue pour remettre les vertèbres dans la position souhaitée.

Un tel dispositif permet de soulager la colonne vertébrale. Son redressement et la présence de tiges solides de part et d'autre, auxquelles les vertèbres s'accrochent, reprennent en effet les contraintes en pression exercées sur les vertèbres déformées ou abîmées.

Avec un tel dispositif il est souvent utile de prévoir un système permettant de relier entre elles les deux tiges parallèles ou sensiblement parallèles ce qui permet de mieux rigidifier et consolider l'ensemble.

L'invention concerne un système de ce type.

On connaît déjà des dispositifs de fixation postérieure du rachis par vis pédiculaires comprenant au moins deux barres transversales reliant mécaniquement les deux tiges longitudinales parallèles à la colonne vertébrale.

WO 2009/117111 propose par exemple un dispositif de fixation enter elles de deux tiges de maintien rachidien. Le dispositif comprend un élément transversal muni à chaque extrémité d'une tête de fixation sur la tige correspondante. Chaque tête comprend une pièce amovible en cornière, de blocage de la tige introduite dans un évidement solidaire de l'élément transversal.

Chaque tête de fixation comporte de plus des chambres ajourées de pénétration d'une extrémité de l'élément autorisant un débattement horizontal et vertical de l'élément permettant de s'ajuster aux creux et bosses de la colonne.

Un tel dispositif est fastidieux à mettre en place et nécessite de nombreuses petites pièces à visser.

On connaît également des systèmes où la fixation sur les vertèbres se fait elle-même par l'intermédiaire des têtes de fixation de l'élément transversal.

De tels systèmes n'ont aucune souplesse et ne peuvent fonctionner que sur des cas simples.

Les documents US 2005/0228377, US 2007/0276367, US 2008/0109039, US 2008/0306538 et US 2005/0090821 décrivent des systèmes ajustables comprenant des têtes intermédiaires autorisant des degrés de liberté entre branches et des fixations sur les tiges par vissage/clipsage. Ces systèmes présentent des jeux importants au moment du montage.

On connaît aussi (US 2007/0043365) un dispositif de fixation de vis pédiculaire dans des vertèbres adjacentes, sur une tige par blocage/vissage, mais en excluant toute notion de consolidage transversal.

De façon générale les systèmes de l'art antérieur ne sont pas naturellement stables, sont fastidieux à mettre en oeuvre, sont encombrants et ne permettent pas un serrage réellement homogène de la pièce de fixation sur la tige. La présente invention propose un système de liaison entre deux tiges cylindriques longitudinales de maintien rachidien selon la revendication indépendante 1. Des réalisations avantageuses de l'invention sont décrites dans les revendications dépendantes.

La présente invention vise à fournir un système de liaison répondant mieux que ceux antérieurement connus aux exigences de la pratique, notamment en ce qu'elle autorise une tenue immédiate de l'élément de liaison sur les tiges par clipsage ou clippage, ce qui rend le système autostable, facilitant la pose par le chirurgien, en ce qu'il présente un encombrement très faible au-dessus de la tige, de la hauteur d'une tête de vis pédiculaire, ce qui évite ainsi des douleurs musculaires au patient, et en ce qu'il permet un serrage parfaitement homogène de la pièce de fixation sur la tige, sur une surface importante de cette dernière.

Le système selon l'invention est ainsi particulièrement indiqué pour les opérations de réduction des déformations (scolioses) notamment chez les patients jeunes et/ou menus de zone thoracique de faible volume.

Dans ce but, l'invention propose essentiellement un système de liaison entre deux tiges cylindriques longitudinales de maintien rachidien, comportant au moins un élément allongé transversal muni à chacune de ses extrémités d'une tête de fixation sur une tige correspondante, caractérisé en ce que au moins une des dites têtes de fixation comprend un évidement semi-cylindrique de clippage avec la tige présentant une lèvre supérieure coiffant le dessus de la tige et une lèvre inférieure opposée radialement, et en ce que ladite tête comporte des moyens de déformation par flexion de ladite lèvre inférieure entre une position de connexion et une position de blocage sur la tige.

L'évidement semi cylindrique de clippage permet notamment un serrage homogène sur la tige, la surface de contact avec la tige étant en effet la surface interne de l'évidement de section égale ou sensiblement égale à la moitié de la circonférence de la tige.

Dans des modes de réalisation avantageux, on a de plus recours à l'une et/ou à l'autre des dispositions suivantes :
- la tête est formée d'un corps comprenant d'un côté ledit évidement et de l'autre côté les moyens de déformation de la lèvre inférieure ;
- les moyens de déformation comportent une zone fendue comprenant une languette inférieure solidaire de la lèvre inférieure et séparée du reste du corps par une fente, ledit reste du corps étant solidaire de l'élément allongé, et des moyens d'écartement de ladite languette du reste du corps pour déformer ladite lèvre inférieure entre sa position de connexion et sa position de blocage ;
- les moyens d'écartement sont formés d'une vis de serrage prenant appui d'un côté sur la face intérieure de la fente de la languette et coopérant de l'autre côté avec un filetage solidaire du reste du corps ;
- les deux têtes de fixation présentent une configuration identique ;
- les évidements sont dirigés du même coté par rapport à une direction transversale déterminée ;
- l'autre tête comprend un évidement cylindrique de passage d'une desdites tiges cylindriques propre à coopérer à frottement doux avec ladite tige ;
- l'élément allongé transversal comprend deux branches chacune solidaire d'une tête correspondante, agencées pour coulisser l'une par rapport à l'autre et des moyens de blocage dans les plans frontal et horizontal de l'une des branches sur l'autre par une vis de blocage ;
- la vis de blocage coopère avec un pas de vis appartenant à une tête intermédiaire solidaire d'une des deux branches, ladite tête intermédiaire comprenant un évidement de passage de l'extrémité de l'autre branche, ledit évidement autorisant un jeu dans les plans frontal et horizontal, de l'extrémité par rapport à ladite tête intermédiaire, l'extrémité de la vis étant quant-à-elle agencée pour s'appuyer sur, et bloquer l'autre branche lorsqu'elle est mise en compression par vissage sur cette dernière.

L'invention propose également un dispositif comprenant au moins deux tiges cylindriques et au moins un système tel que décrit ci-avant.

L'invention sera mieux comprise à la lecture de la description qui suit de modes de réalisation donnés à titre d'exemples non limitatifs. La description se réfère aux dessins qui l'accompagnent dans lesquels :
La figure 1 est une vue de face d'une portion de colonne vertébrale munie d'un système de liaison entre deux tiges selon un premier mode de réalisation de l'invention.
La figure 2 est une vue en perspective partielle de deux tiges munies de deux systèmes de liaison, selon deux modes de réalisation de l'invention.
La figure 3 est une vue en coupe selon III-III d'un des systèmes de la figure 2.
La figure 4 est une vue en coupe selon IV-IV de la tête de fixation du dispositif de la figure 3.

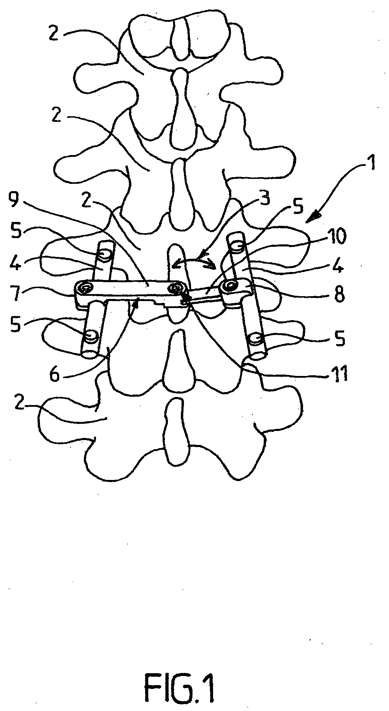

La figure 1 montre une portion 1 de colonne vertébrale comprenant des vertèbres 2 et un système 3 de liaison selon un mode de réalisation de l'invention, entre deux tiges cylindriques 4.

Les tiges sont fixées de façon connue en elle-même par des vis pédiculaires 5 sur les vertèbres en vis à vis.

Le système 3 comprend un élément allongé 6 transversal muni à chacune de ses extrémités d'une tête 7 et 8 de fixation sur une tige 2 correspondante.

L'élément allongé 6 comprend deux branches 9 et 10 solidaires l'une de l'autre, agencées pour coulisser l'une par rapport à l'autre et des moyens 11 de blocage dans les plans frontal (parallèle aux tiges) et horizontal (perpendiculaire aux tiges) de l'une des branches 9 par rapport à l'autre 10.

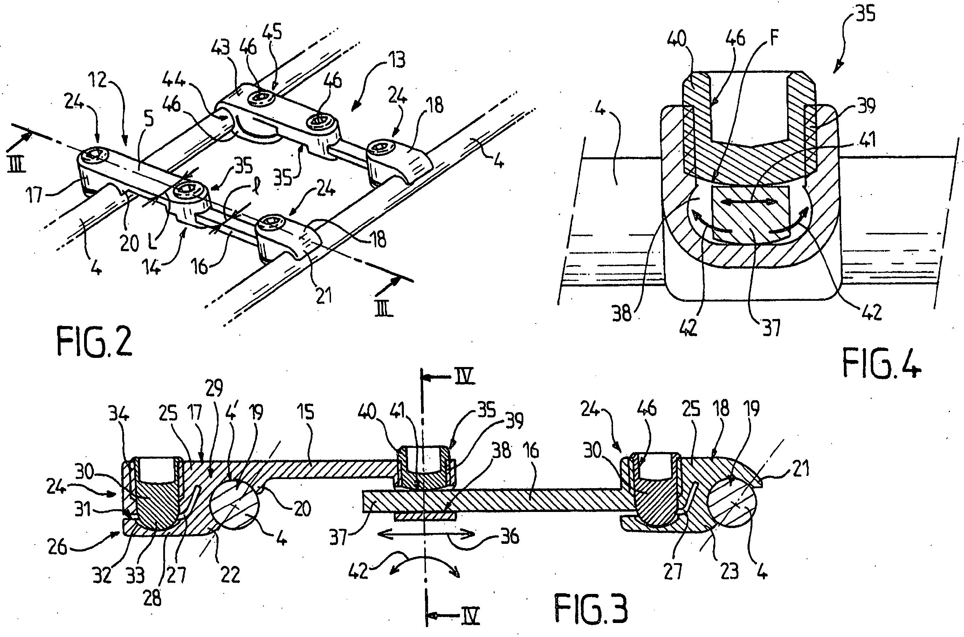

La figure 2 montre en perspective deux modes de réalisation 12 et 13 du système selon l'invention.

Dans la suite du texte on utilisera les mêmes numéros de référence pour désigner les mêmes éléments.

Le système 12 comprend un élément allongé 14 transversal formé de deux branches 15 et 16, à savoir une première branche 15 comprenant une tête 17 de fixation sur la tige 4 et une deuxième branche 16 comprenant de l'autre côté une tête 18 de fixation sur l'autre tige 4.

Chaque tête de fixation (cf. figure 3) comprend un évidement semi-cylindrique 19 de clippage avec la tige présentant une lèvre supérieure 20, 21 coiffant partiellement le dessus 4' de la tige et une lèvre inférieure 22, 23 opposée radialement ou sensiblement radialement.

Les évidements 19 et leurs lèvres sont disposés pour permettre un clippage de la pièce sur une tige 4 correspondante, du fait d'une possibilité de légère déformation de la lèvre sur la tige rigide.

Selon un aspect de l'invention les lèvres inférieures 22, 23 peuvent être déformées.

Plus précisément chaque tête de fixation comporte des moyens 24 de déformation par flexion de la lèvre 22, 23 entre une position, libre, de connexion permettant un clippage facile et une position de blocage en pression sur la tige.

Pour ce faire la tête est formée par un corps 25 comprenant d'un côté l'évidement 19 et de l'autre côté les moyens 24 de déformation.

Ceux-ci comprennent une zone 26 fendue comprenant une fente 27 transversale de section en L ou en crosse de hockey séparant une portion 28 en forme de languette plate inférieure et le reste 29 du corps comprenant d'un côté l'évidement 19 solidaire de la branche 15, du même côté pour une des têtes 17 et de l'autre côté pour la tête 18, et comprenant de l'autre côté, des moyens d'écartement de ladite languette du reste du corps.

Ces moyens d'écartement sont formés par une vis 30 de gros diamètre, par exemple 1 cm, à pointe arrondie, ladite pointe arrondie prenant appui sur la face intérieure 31, par exemple présentant un évidement 32 en forme de coupelle, de réception de l'extrémité 33 par exemple sphérique de la vis 30 et coopérant de l'autre côté avec un filetage 34 solidaire du reste 29 du corps.

La branche 15 présente une portion allongée d'une première largeur L par exemple parallélépipédique et se termine, de l'autre côté de la tête de fixation 17 sur la base 4, par une tête intermédiaire 35 par exemple en forme de pion cylindrique, agencée pour permettre à la branche 16 de coulisser par rapport à la branche 15 dans le sens transversal (flèche 36) par rapport aux tiges 4.

La branche 16 est quant-à-elle terminée d'un côté par la tête de fixation 18.

Elle présente une portion allongée, par exemple parallélépipédique à fond partiellement cylindrique ou à tout le moins arrondi, concave (cf figure 4), d'une seconde largeur ℓ < L et est reliée de l'autre côté au reste du corps 29 de la tête 18 à une portion située du côté opposé à la tige 4 par rapport à la vis 30 d'écartement.

L'autre extrémité 37 de la portion allongée passe par un évidement 38 de la tête intermédiaire 35, avec lequel il coopère à frottement.

La tête 35 comporte un orifice fileté 39 et une vis 40 de blocage de même type que la vis d'écartement 30, munie d'une extrémité 41 sphérique qui va autoriser une flexibilité de la branche 16 par rapport à la branche 15 selon la flèche 42 dans le plan vertical transversal.

On va décrire plus précisément la tête 35 en référence à la figure 4.

La tête 35 est évidée en partie basse par un évidement 38 de section sensiblement ovale ou en forme de cercle écrasé, plus large, par exemple une fois et demi plus large que la largeur de la branche ℓ, ce qui va permettre un déplacement latéral (flèche 41) de ladite branche 16 par rapport à la tête 35 solidaire de la branche 15, et une possibilité de pivotement autour du sens axial transversal des branches (flèche 42).

De même le fond arrondi F de la vis 40 autorise le débattement angulaire dans le plan perpendiculaire aux branches.

La jonction entre les deux branches permet dès lors de compenser les écarts angulaires possibles entre les deux tiges d'union d'une part et dans le plan parallèle aux vertèbres d'autre part en autorisant un léger vrillage d'un branche par rapport à l'autre.

La figure 2 montre un autre mode de réalisation du système 13 de l'invention pour lequel la tête de fixation 43 sur l'une des tiges est différente.

Ici la tête 43 comprend un orifice ou évidement 44 cylindrique de passage de la tige qui sera donc introduite au préalable, des moyens 45 de même compression de la partie inférieure 46 de la bordure de l'orifice, du type de ceux décrits en référence aux moyens 24, étant prévus, avec fente, languette et vis correspondante.

On va maintenant décrire la mise en place d'un dispositif utilisant un système selon le mode de réalisation de l'invention en référence aux figures 1 et 3.

Après ouverture de la portion dorsale du patient, pour accéder aux vertèbres à redresser, le chirurgien met en place les tiges en les fixant de façon connue en elle-même, par exemple en vissant les vis pédiculaires dans les extrémités des vertèbres concernées.

Il réalise ensuite de façon extrêmement simple et rapide la mise en place du système de liaison par exemple en titane ou en matériau biocompatible dans des dimensions autorisant une certaine souplesse (flexibilité).

La tête centrale étant mobile (vis 40 dévissée) il vient clipper le système qui a alors les degrés de liberté nécessaires dans le plan frontal et sagittal (flèches 36 et 42) pour autoriser le clippage de part et d'autre sans contraintes.

Puis il vient visser par le dessus les vis 30 de fixation sur les tiges, qui comporte par exemple des têtes avec orifice à pas hexagonaux 46, pour vissage de façon connue en elle-même.

Cela permet d'assurer un blocage définitif sur les tiges 4.

Enfin l'ensemble est rigidifié par ajustage et vissage de la vis centrale 40 de la tête 35.

Le chirurgien place ensuite autant de systèmes 3 de rigidification qu'il estime nécessaire.

Avantageusement le système est en alliage de titane, mais il peut aussi être fabriqué dans d'autres matériaux bio-compatibles possédant de bonnes caractéristiques d'élasticité.

Comme il va de soi et comme il résulte également de ce qui précède, la présente invention n'est pas limitée aux modes de réalisation plus particulièrement décrits. Elle en embrasse au contraire toutes les variantes et notamment celles où les branches sont cylindriques, celles où la tête intermédiaire est amovible et/ou celles où les deux têtes de fixation sont identiques mais situées entre les deux tiges, dans un sens opposé l'une par rapport à l'autre.

## Revendications

1. Système (3, 12, 13) de liaison entre deux tiges (4) cylindriques longitudinales de maintien rachidien, comportant au moins un élément allongé transversal (6, 14) muni à chacune de ses extrémités d'une tête (7, 8 ; 17, 18 ; 43) de fixation sur une tige correspondante, chacune des dites têtes (7, 8 ; 17, 18) de fixation comprenant un évidement (19) semi-cylindrique de clippage avec la tige (4) présentant une lèvre supérieure (20, 21) coiffant le dessus de la tige et une lèvre inférieure (22, 23) opposée radialement, ladite tête comportant des moyens (24) de déformation par flexion de ladite lèvre inférieure (22, 23) entre une position de connexion et une position de blocage sur la tige, chaque tête de fixation (17, 18) étant formée d'un corps (25) comprenant d'un côté l'évidement (19) et de l'autre côté les moyens (24) de déformation de la lèvre inférieure, l'élément allongé transversal comprenant deux branches (15, 16) chacune solidaire d'une tête correspondante (17, 18) agencées pour coulisser l'une par rapport à l'autre et des moyens (35) de blocage de l'une des branches (15) sur l'autre (16) par une vis (40) de blocage,
ladite vis de blocage (40) coopérant avec un pas de vis (39) appartenant à une tête intermédiaire (35) solidaire de l'une des branches, comprenant un évidement (38) de passage de l'extrémité (37) de l'autre branche (16),
**caractérisé en ce que** la tête intermédiaire est formée d'une pièce avec l'une des deux branches,
**en ce que** ledit évidement (38) est de section sensiblement ovale ou en forme de cercle écrasé de plus grande largeur que la largeur de ladite autre branche et autorisant un jeu dans les plans frontal et horizontal, de l'extrémité par rapport à ladite tête intermédiaire,
**en ce que** l'extrémité de la vis présente un fond arrondi F prenant appui sur le dessus de l'extrémité (37) de l'autre branche, de blocage de ladite autre branche (16) dans l'évidement (38) par vissage de ladite vis (40) dans un orifice fileté (39) de la tête intermédiaire sur cette dernière,et **en ce que** les moyens de déformation comportent une zone (26) fendue comprenant une languette (28) inférieure solidaire de la lèvre inférieure (22, 23) et séparée du reste du corps par une fente (27), ledit reste (29) du corps étant solidaire de l'élément allongé, et des moyens d'écartement de ladite languette du reste du corps pour déformer ladite lèvre inférieure entre sa position de connexion et sa position de blocage.

2. Système selon la revendication 1, **caractérisé en ce que** les moyens (30, 31) d'écartement sont formés d'une vis (30) de serrage prenant appui d'un côté sur la face intérieure (31) de la fente de la languette et coopérant de l'autre côté avec un filetage (34) solidaire du reste (29) du corps.

3. Système selon l'une quelconque des revendications précédentes, **caractérisé en ce que** les deux têtes (17, 18) de fixation présentent une configuration identique.

4. Système selon l'une quelconque des revendications 1 à 3, **caractérisé en ce que** l'autre tête comprend un évidement (44) cylindrique de passage d'une desdites tiges (4) cylindriques propre à coopérer à frottement doux avec ladite tige.

5. Système selon la revendication 4, **caractérisé en ce que** la branche (16) présente une portion allongée parallélépipédique à fond concave.

6. Système selon l'une quelconque des revendications précédentes **caractérisé en ce que** les évidements (19) sont chacun situés du même côté de leur tête de fixation par rapport à une direction transversale déterminée.

7. Dispositif, **caractérisé en ce qu'**il comporte deux tiges (4) cylindriques et au moins un système (3, 12, 13) selon l'une quelconque des revendications précédentes.

## Patentansprüche

1. System (3, 12, 13) für eine Verbindung zwischen zylindrischen länglichen Stiften (4) zur Wirbelstützung, umfassend wenigstens ein längliches Querelement (6, 14), das an jedem Ende mit einem Kopf (7, 8; 17, 18; 43) zur Befestigung an einem entsprechenden Stift versehen ist, wobei jeder der Befestigungsköpfe (7, 8; 17, 18) eine halbzylindrischen Aussparung (19) zum Verclippen mit dem Stift (4)aufweist, welche eine obere Lippe (20, 21) aufweist, die oben auf den Stift aufgesetzt ist, und eine untere Lippe (22, 23), die radial entgegengesetzt ist, wobei der Kopf Mittel (24) zur Verformung der unteren Lippe (22, 23) durch Biegung zwischen einer Verbindungsstellung und einer Blockierstellung an dem Stift, wobei jeder Befestigungskopf (17, 18) durch einen Körper (25) gebildet ist, der auf einer Seite die Aussparung (19) aufweist und auf der anderen Seite die Mittel (24) zur Verformung der unteren Lippe aufweist, wobei das längliche Querelement zwei Schenkel (15, 16) aufweist, die jeweils mit einem entsprechenden Kopf (17, 18) fest verbunden sind und angeordnet sind, um relativ zueinander zu gleiten, und Mittel (35) zur Blockierung eines der Schenkel (15) an dem anderen (16) durch eine Sicherungsschraube (40), wobei die Sicherungsschraube (40) mit einem Gewindegang (39) zusammenwirkt, der zu einem Zwischenkopf (35) gehört, der mit einem der Schenkel fest verbunden ist, umfassend eine Aussparung (38) für die Durchführung des Endes (37) des anderen Schenkels (16),
**dadurch gekennzeichnet,**
**dass** der Zwischenkopf einstückig mit einem der beiden Schenkel ausgebildet ist,
die Aussparung (38) einen im Wesentlichen ovalen Querschnitt aufweist oder einen Querschnitt in Form eines flachgedrückten Kreises, dessen Breite größer ist als die Breite des anderen Schenkels und der in der frontalen und horizontalen Ebene ermöglicht ein Spiel des Endes relativ zu dem Zwischenkopf ermöglicht, und das Ende der Schraube einen gerundeten, oben auf das Ende (37) des anderen Schenkels zur Anlage kommenden Boden F aufweist zur Blockierung des anderen Schenkels (16) in der Aussparung (38) durch Verschraubung der Schraube (40) in einer mit einem Gewinde versehenen Öffnung (39) des Zwischenkopfes auf diesen, und die Verformungsmittel einen geschlitzten Bereich (26) aufweisen, der eine untere Lasche (28) aufweist, die mit der unteren Lippe (22, 23) fest verbunden ist und vom restlichen Körper durch einen Schlitz (27) getrennt ist, wobei der Rest (29) des Körpers mit dem länglichen Element fest verbunden ist, und Mittel zur Beabstandung der Lasche vom restlichen Körper, um die untere Lippe zwischen ihrer Verbindungsstellung und ihrer Blockierstellung zu verformen.

2. System nach Anspruch 1,
**dadurch gekennzeichnet,**
**dass** die Beabstandungsmittel (30, 31) durch eine Klemmschraube (30) gebildet sind, die sich einerseits an der Innenseite (31) des Schlitzes der Lasche abstützt und andererseits mit einem Gewinde (34) zusammenwirkt, das mit dem Rest (29) des Körpers fest verbunden ist.

3. System nach einem der vorhergehenden Ansprüche,
**dadurch gekennzeichnet,**
**dass** die zwei Befestigungsköpfe (17, 18) identisch ausgebildet sind.

4. System nach einem der Ansprüche 1 bis 3,
**dadurch gekennzeichnet,**
**dass** der andere Kopf eine zylindrische Aussparung (44) für die Durchführung eines der zylindrischen Stifte (4) aufweist, die geeignet ist, mit dem Stift in leichter Reibwirkung zu stehen.

5. System nach Anspruch 4,
**dadurch gekennzeichnet,**
**dass** der Schenkel (16) einen quaderförmigen länglichen Abschnitt mit konkavem Boden aufweist.

6. System nach einem der vorhergehenden Ansprüche,
**dadurch gekennzeichnet,**
**dass** die Aussparungen (19) jeweils auf derselben Seite ihres Befestigungskopfes relativ zu einer bestimmten Querrichtung angeordnet sind.

7. Vorrichtung,
**dadurch gekennzeichnet,**
**dass** sie zwei zylindrische Stifte (4) und mindestens ein System (3, 12, 13) nach einem beliebigen der vorhergehenden Ansprüche aufweist.

## Claims

1. System (3, 12, 13) for connection between two longitudinal cylindrical rods (4) for spinal support, comprising at least one transverse elongated element (6, 14) fitted at each of its ends with a head (7, 8; 17, 18; 43) for fixation on a corresponding rod, each of said fixing heads (7, 8; 17, 18) comprising a semi-cylindrical recess (19) for clipping to the rod (4) exhibiting an upper lip (20, 21) fitting on the top of the rod and a lower lip (22, 23) which is radially opposed, said head comprising means (24) for deformation by flexion of said lower lip (22, 23) between a position for connection and a position for locking on the rod, each fixation head (17, 18) being formed of a body (25) comprising on one side the recess (19) and on the other side the means (24) for deformation of the lower lip, the transverse elongated element comprising two limbs (15, 16) each integral with a corresponding head (17, 18) arranged to slide one in relation to the other and means (35) for locking of one of the limbs (15) on the other (16) by a locking screw (40),
said locking screw (40) co-operating with a screw thread (39) belonging to an intermediate head (35) integral with one of the limbs, comprising a recess (38) for passage of the end (37) of the other limb (16),
**characterised in that** the intermediate head is formed in one piece with one of the two limbs,
**in that** said recess (38) is of substantially oval section or in the form of a squashed circle of greater width than the width of said other limb and allowing a play in the frontal and horizontal planes of the end in relation to said intermediate head,
**in that** the end of the screw exhibits a rounded bottom F bearing on the top of the end (37) of the other limb, for locking said other limb (16) in the recess (38) by screwing of said screw (40) in a threaded hole (39) in the intermediate head on the latter,
and **in that** the means of deformation comprise a split zone (26) comprising a lower tongue (28) integral with the lower lip (22, 23) and separated from the remainder of the body by a slot (27), said remainder (29) of the body being integral with the elongated element, and means for distancing said tongue from the remainder of the body to deform said lower lip between its position for connection and its position for locking.

2. System according to claim 1, **characterised in that** the means (30, 31) for distancing are formed by a clamping screw (30) bearing on one side on the internal face (31) of the slot of the tongue and co-operating on the other side with a thread (34) integral with the remainder (29) of the body.

3. System according to any one of the preceding claims, **characterised in that** the two fixation heads (17, 18) exhibit an identical configuration.

4. System according to any one of claims 1 to 3, **characterised in that** the other head comprises a cylindrical recess (44) for passage of one of said cylindrical rods (4) suitable for co-operating with mild friction with said rod.

5. System according to claim 4, **characterised in that** the limb (16) exhibits a parallelepiped elongated portion with a concave bottom.

6. System according to any one of the preceding claims, **characterised in that** the recesses (19) are each situated on the same side of their fixation head in relation to a given transverse direction.

7. Device, **characterised in that** it comprises two cylindrical rods (4) and at least one system (3, 12, 13) according to any one of the preceding claims.
